# EUROPEAN PATENT SPECIFICATION

(11) **EP 3 738 647 B1**
(45) Date of publication and mention of the grant of the patent: **15.05.2024**
(21) Application number: 20171830.1
(22) Date of filing: 28.04.2020
(51) Int. Cl.: A61B 5/24, A61N 1/36, A61N 1/04, A61B 5/00

(54) **NERVE STIMULATION MEASUREMENT DEVICE, NERVE STIMULATION MEASUREMENT METHOD, AND NERVE STIMULATION MEASUREMENT PROGRAM**
NERVENSTIMULATIONSMESSVORRICHTUNG, NERVENSTIMULATIONSMESSVERFAHREN UND NERVENSTIMULATIONSMESSPROGRAMM
DISPOSITIF DE MESURE DE STIMULATION NERVEUSE, PROCÉDÉ DE MESURE DE STIMULATION NERVEUSE ET PROGRAMME DE MESURE DE STIMULATION NERVEUSE

(30) Priority: 14.05.2019 JP 2019091281
(43) Date of publication of application: 18.11.2020
(73) Proprietor: Nihon Kohden Corporation, Shinjuku-ku Tokyo (JP)
(72) Inventor: KAIAMI, Takashi, Tokorozawa-shi, Saitama (JP); MOTOGI, Jun, Tokorozawa-shi, Saitama (JP)
(74) Representative: Grünecker Patent- und Rechtsanwälte PartG mbB

(56) References cited:
- WO-A1-2013/074809
- WO-A1-2016/146758
- US-A- 5 806 522
- US-A1- 2019 001 135

## Description

### TECHNICAL FIELD

The presently disclosed subject matter relates to a nerve stimulation measurement device, a nerve stimulation measurement method, and a nerve stimulation measurement program.

### BACKGROUND ART

A pain sensory nerve stimulation apparatus is known in which a current having a current value that is manually set is flown through an electrode bonded to a subject to apply electrical stimulation to the subject, and electrical stimulation that is sensed by the subject is measured (for example, Patent Literature 1).
WO 2013/074809 A1 discloses an apparatus and method for relieving pain using transcutaneous electrical nerve stimulation.

### CITATION LIST

### PATENT LITERATURE

[Patent Literature 1] JP2010-88802

### SUMMARY

### TECHNICAL PROBLEM

In a conventional pain sensory nerve stimulation apparatus, however, the inspector must search the minimum current value by which the subject can sense electrical stimulation while manually changing the current for applying electrical stimulation, and therefore the inspector takes a lot of trouble. The timing when electrical stimulation is applied varies according to the inspector, and therefore a measurement result depends on the inspector. In a conventional pain sensory nerve stimulation apparatus, therefore, the measurement is cumbersome, and a reproducible measurement result is hardly obtained.

The presently disclosed subject matter has been conducted in order to solve the problems of such a conventional pain sensory nerve stimulation apparatus. It is an object of the presently disclosed subject matter to provide a nerve stimulation measurement device, nerve stimulation measurement method, and nerve stimulation measurement program in which a reproducible measurement result can be obtained by a simple configuration.

### SOLUTION TO PROBLEM

The invention provides a nerve stimulation measurement device according to appended claim 1, a nerve stimulation measurement method according to appended claim 10 and a nerve stimulation measurement program according to appended claim 11. Advantageous embodiments are set out in the dependent claims.

### ADVANTAGEOUS EFFECTS

According to the nerve stimulation measurement device, nerve stimulation measurement method, and nerve stimulation measurement program of the presently disclosed subject matter, a reproducible measurement result can be obtained by a simple configuration.

### BRIEF DESCRIPTION OF DRAWINGS

FIG. 1 is an external view of a nerve stimulation measurement device of an embodiment during measurement.
FIG. 2 is a block diagram of a control system of the nerve stimulation measurement device of the embodiment.
FIG. 3 is a diagram of a controlling section of the nerve stimulation measurement device of the embodiment.
FIG. 4 is an operation flowchart of the nerve stimulation measurement device of the embodiment.
FIG. 5 is an operation flowchart of the nerve stimulation measurement device of the embodiment.
FIG. 6 is an operation flowchart of the nerve stimulation measurement device of the embodiment.
FIG. 7 is a view illustrating Processing mode 1 in the nerve stimulation measurement device of the embodiment.
FIG. 8 is a view illustrating Processing mode 2 in the nerve stimulation measurement device of the embodiment.
FIG. 9 is a view illustrating Processing mode 3 in the nerve stimulation measurement device of the embodiment.
FIG. 10 is a view illustrating Processing mode 4 in the nerve stimulation measurement device of the embodiment.

### DESCRIPTION OF EMBODIMENTS

Hereinafter, an embodiment of the nerve stimulation measurement device, nerve stimulation measurement method, and nerve stimulation measurement program of the presently disclosed subject matter will be described.

### <Configuration of nerve stimulation measurement device>

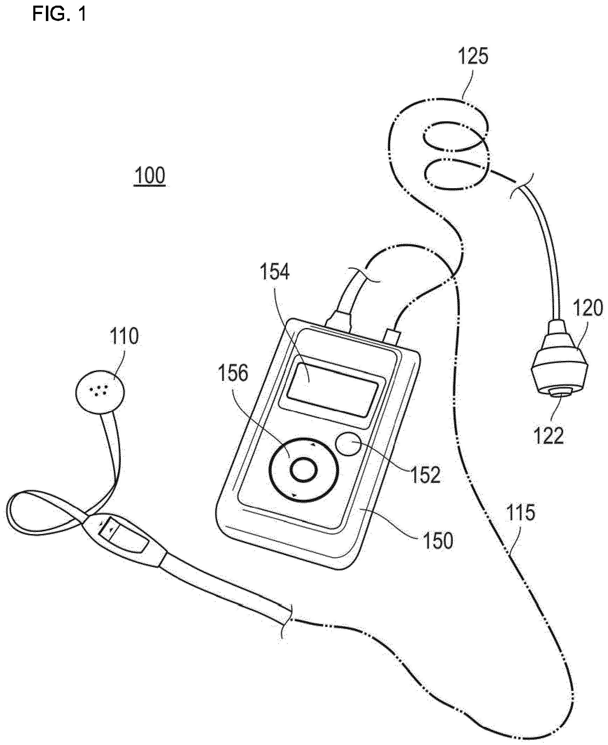

FIG. 1 is an external view of the nerve stimulation measurement device of the embodiment during measurement. The nerve stimulation measurement device 100 may include a responding section 120 and a body unit 150. The body unit 150 is configured so as to be attachable to and detachable from an electrode section 110 that is used during measurement.

The electrode section 110 that is connected to the body unit 150 during measurement is attached to a portion of a subject that easily senses electrical stimulation, such as the top of the hand or foot of the subject. The electrode section 110 is connected to the body unit 150 through a cord 115. The electrode section 110 applies electrical stimulation by using a stimulation current that is supplied from the body unit 150.

The responding section 120 is used for performing a response operation in the case where the subject senses the electrical stimulation. The responding section 120 is connected to the body unit 150 through a cord 125. The responding section 120 may include a response button 122. When the subject senses the electrical stimulation, the response button 122 is pressed by the subject. When the response button 122 is pressed, the body unit 150 confirms a response of the subject due to the stimulation current.

The body unit 150 may include a start switch 152, a displaying section 154, and an inputting section 156. The start switch 152 is pressed when the measurement using the nerve stimulation measurement device 100 is to be started. When the start switch 152 is pressed, a series of processes from the supply of the stimulation current to the electrode section 110 to the obtaining of a sensitivity threshold (a stimulation current from which the subject senses the electrical stimulation, and which will be described later in detail) are automatically performed. Although, in the embodiment, the start switch 152 is disposed in a part of the body unit 150, the start switch may be displayed on the displaying section 154 that will be described later, and the displayed start switch 152 may be pressed.

The displaying section 154 displays the above-described sensitivity threshold that is obtained by the body unit 150, or information indicating that the measurement is impossible. The display contents will be described later in detail. In the embodiment, an LCD display or organic EL display that is usually used may be employed as the displaying section 154.

The inputting section 156 is used for inputting values that are necessary for measurement, such as: the initial current value of the stimulation current; a predetermined increment of the stimulation current that stepwise increases; a predetermined decrement of the stimulation current that stepwise decreases; the response waiting time period; the predetermined number of sessions; the predetermined number of valid sessions; and the predetermined range of the effective current, to a storage section that is disposed in the body unit 150. The predetermined number of valid sessions is the number of sessions that are required for obtaining the sensitivity threshold. The disposition of the inputting section 156 enables the operator to freely set the values that are necessary for measurement. Although, in the embodiment, the inputting section 156 is disposed in a part of the body unit 150, an input port that can be connected to an external computer may be used as the inputting section 156. In this case, the values that are necessary for measurement are input from the external computer through the input port. The values that are necessary for measurement will be described later in detail.

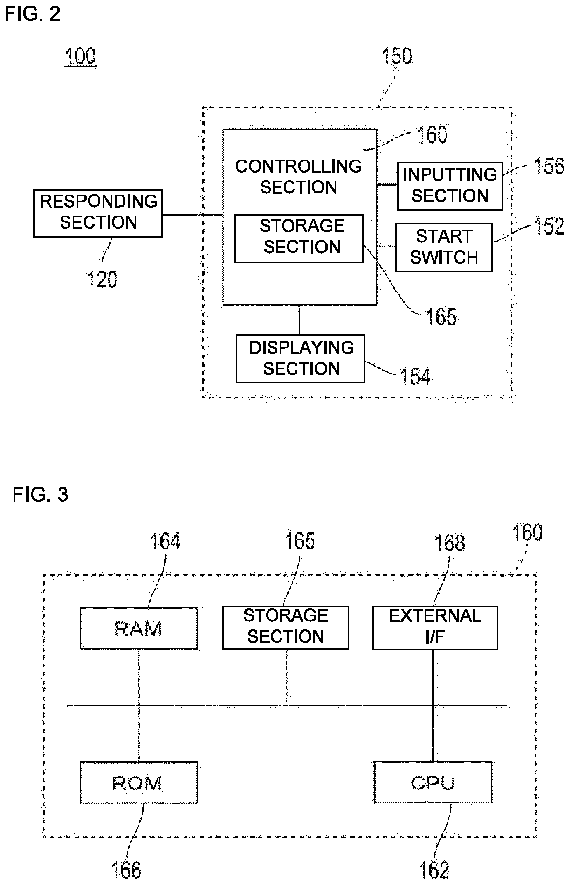

FIG. 2 is a block diagram of a control system of the nerve stimulation measurement device of the embodiment. The nerve stimulation measurement device 100 has the responding section 120 and the body unit 150. The body unit 150 has the start switch 152, the displaying section 154, the inputting section 156, and a controlling section 160. The controlling section 160 has the storage section 165.

The responding section 120, the body unit 150, the start switch 152, the displaying section 154, and the inputting section 156 are configured as described above.

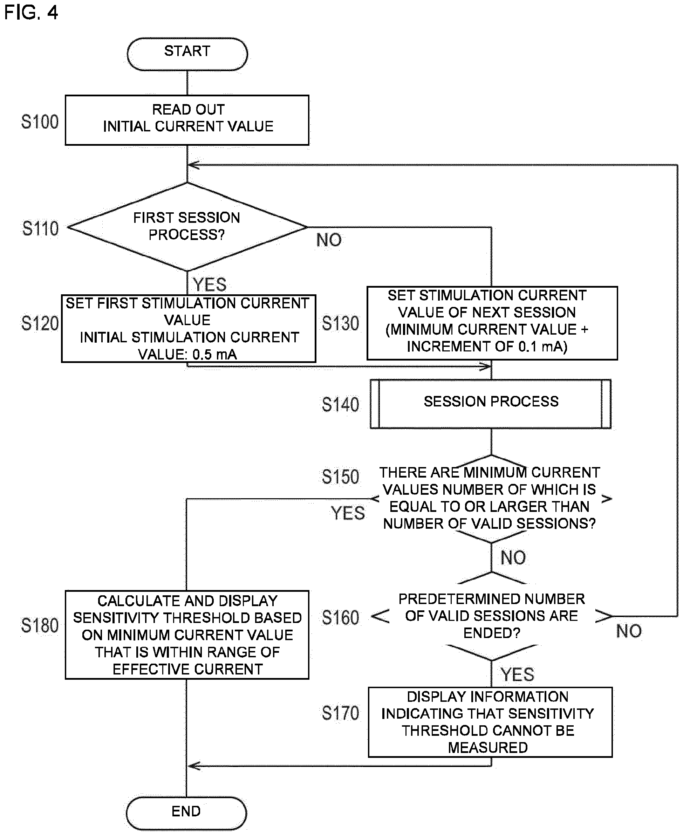

The controlling section 160 supplies the stimulation current that stepwise increases or decreases, to the electrode section 110, and obtains the sensitivity threshold that is a stimulation current from which the subject senses the electrical stimulation, from the situation of a response of the responding section 120 to each stepwise supply. In the case where the stimulation current that stepwise increases is to be supplied to the electrode section 110, the controlling section 160 stepwise increases the stimulation current according to the predetermined increment that is stored in the storage section 165, and, in the case where the stimulation current that stepwise decreases is to be supplied to the electrode section 110, the controlling section 160 stepwise decreases the stimulation current according to the predetermined decrement that is stored in the storage section 165. The operation of the controlling section 160 will be described later in detail with reference to FIG. 4 and subsequent figures.

The storage section 165 stores the values that are supplied from the inputting section 156, and that are necessary for measurement, such as: the initial current value of the stimulation current; the increment of the stimulation current that stepwise increases; the decrement of the stimulation current that stepwise decreases; the response waiting time period; the predetermined number of sessions; the predetermined number of valid sessions; and the predetermined range of the effective current. The storage section 165 further stores the minimum current value and sensitivity threshold in each session process that are obtained by the controlling section 160.

FIG. 3 is a diagram of the controlling section of the nerve stimulation measurement device of the embodiment. The controlling section 160 may include: a CPU 162 that performs a series of processes for nerve stimulation measurement; a RAM 164 that temporarily stores results of the series of processes performed by the CPU 162; the storage section 165 that stores values that are necessary for measurement; a ROM 166 that stores a nerve stimulation measurement program that is to be executed for nerve stimulation measurement by the CPU 162; and an external interface (external I/F) 168 that communicates with the external computer. The controlling section 160 having the configuration of FIG. 3 functions as a computer.

### <Operation of nerve stimulation measurement device>

FIGS. 4 to 6 are operation flowcharts of the nerve stimulation measurement device of the embodiment. The operation of the nerve stimulation measurement device 100 will be described in detail with reference to the operation flowcharts. The operation flowcharts of FIGS. 4 to 6 illustrate also the procedure of the nerve stimulation measurement method and nerve stimulation measurement program of the embodiment.

In the operation flowcharts of FIGS. 4 to 6, the process is started when the start switch 152 (see FIGS. 1 and 2) is pressed.

When the start switch 152 is pressed and the process is started, the controlling section 160 reads out the initial current value from the storage section 165 (S100).

The controlling section 160 determines whether the process to be next performed is the first session process or not (S110). If the process is the first session process (S110: YES), the controlling section 160 sets the supplied initial current value as the first stimulation current value, i.e., the initial stimulation current value (S120). In the embodiment, the initial stimulation current value is 0.5 mA. However, the setting of the initial stimulation current value is not limited to the current value. Then, the controlling section 160 performs the first session process (S140).

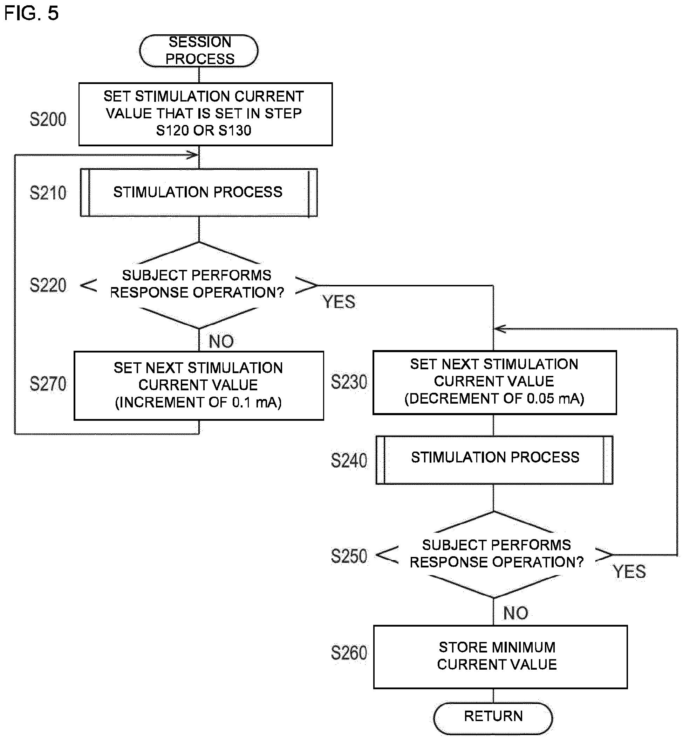

The session process is executed in accordance with the process of the operation flowchart of FIG. 5. The controlling section 160 sets the stimulation current value that is set in step S120 of the operation flowchart of FIG. 4 (S200). In the case of the first session process, the controlling section 160 sets the initial stimulation current value as the stimulation current value. When the setting of the stimulation current value is ended, the controlling section 160 then performs a stimulation process (S210).

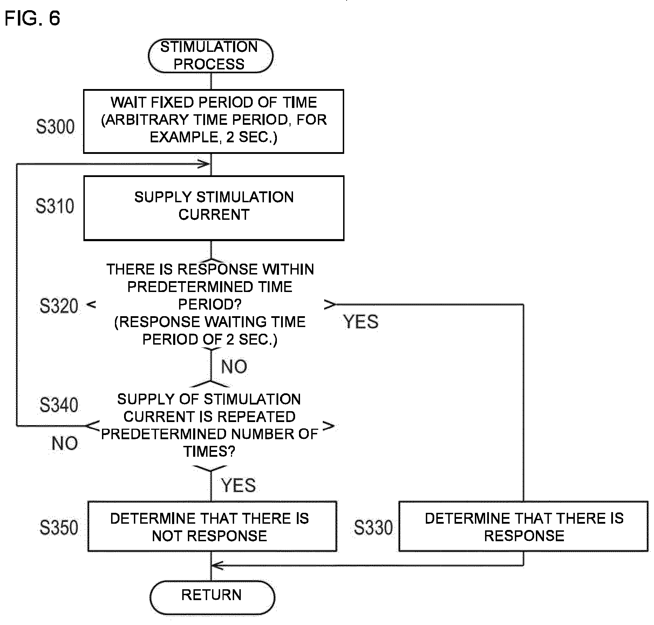

The stimulation process (S210) is executed in accordance with the process of the operation flowchart of FIG. 6. When the stimulation process is started, the controlling section 160 waits a fixed period of time (an arbitrary time period, for example, 2 sec.) (S300), and then supplies the stimulation current to the electrode section 110 (see FIGS. 1 and 2) (S310). In the case of the first session process, the controlling section 160 supplies the stimulation current having the initial stimulation current value to the electrode section 110. The stimulation current is supplied from the electrode section 110 to the subject. When the subject senses the electrical stimulation, the subject responses to the stimulation by pressing the response button 122 of the responding section 120.

The controlling section 160 determines whether there is a response within a predetermined time period or not (S320). In the embodiment, the predetermined time period, i.e., the response waiting time period is 2 sec. However, the waiting time period is not limited to the time period.

If a response operation is performed within the predetermined time period (S320: YES), the controlling section 160 determines that there is a response (S330). By contrast, if a response operation is not performed within the predetermined time period (S320: NO), the controlling section 160 determines whether the supply of the stimulation current is repeated a predetermined number of times or not (S340). The process of step S340 is performed because determination of no response based only on one response confirmation is not desirable in view of ensuring of reliability of the measurement.

If the supply of the stimulation current is not repeated the predetermined number of times (S340: NO), the controlling section 160 repeats the processes of steps S310 and S320 until a response operation is performed by the subject, or the supply of the stimulation current is repeated the predetermined number of times. If the supply of the stimulation current is repeated the predetermined number of times (S340: YES), the controlling section 160 determines that there is not a response (S350).

When the stimulation process of the operation flowchart of FIG. 6 is ended, and either one of the determinations that there is a response and that there is not a response is performed, the control proceeds to step S220 of the operation flowchart of FIG. 5.

Next, the controlling section 160 determines whether the subject performs a response operation or not, from the result of the stimulation process of the operation flowchart of FIG. 6 (S220). If the subject performs a response operation (S220: YES), the controlling section 160 sets the next stimulation current value with reference to the decrement that is stored in the storage section 165, and that is used for the stimulation current that stepwise decreases (S230). In the embodiment, the decrement of the stimulation current is 0.05 mA. However, the decrement of the stimulation current is not limited to the current value. In this case, the initial stimulation current value is 0.5 mA, and the decrement of the stimulation current is 0.05 mA. Therefore, the next stimulation current is set to 0.45 mA.

The controlling section 160 performs the stimulation process of the operation flowchart of FIG. 6 by using the next stimulation current value that is set as described above (S240). In this case, the controlling section 160 checks the presence or absence of a response performed by the subject, while supplying the stimulation current of 0.45 mA to the electrode section 110.

When the stimulation process of the operation flowchart of FIG. 6 is ended, and either one of the determinations that there is a response and that there is not a response is performed, the control proceeds to step S250 of the operation flowchart of FIG. 5.

Next, the controlling section 160 determines whether the subject performs a response operation or not, from the result of the stimulation process of the operation flowchart of FIG. 6 (S250). If the subject performs a response operation (S250: YES), the controlling section 160 sets the next stimulation current value with reference to the decrement that is stored in the storage section 165, and that is used for the stimulation current that stepwise decreases (S230). In this case, the previous stimulation current value is 0.45 mA, and the decrement of the stimulation current is 0.05 mA. Therefore, the next stimulation current is set to 0.4 mA. The processes of steps S230 and S240 are repeated until a response performed by the subject is eliminated in the result of the stimulation process of the operation flowchart of FIG. 6.

The controlling section 160 repeats the processes of steps S230 and S240. If there is no response performed by the subject (S250: NO), the controlling section 160 causes the minimum current value that is the minimum stimulation current obtained immediately before the subject does not perform the response operation, to be stored in the storage section 165 (S260).

The control returns to the process of step S220. If there is no response performed by the subject (S220: NO), the controlling section 160 sets the next stimulation current value with reference to the increment that is stored in the storage section 165, and that is used for the stimulation current that stepwise increases (S270). In the embodiment, the increment of the stimulation current is 0.1 mA. However, the increment of the stimulation current is not limited to the current value. In this case, the initial stimulation current value is 0.5 mA, and the increment of the stimulation current is 0.1 mA. Therefore, the next stimulation current is set to 0.6 mA.

The controlling section 160 performs the stimulation process of the operation flowchart of FIG. 6 by using the next stimulation current value that is set as described above (S210). In this case, the controlling section 160 checks the presence or absence of a response performed by the subject, while supplying the stimulation current of 0.6 mA to the electrode section 110. With respect to the processes of steps S210 and S240 of performing the stimulation process of the operation flowchart of FIG. 6, the process of step S210 is repeated until there is a response performed by the subject, and that of step S240 is repeated until a response performed by the subject is eliminated.

As a result of the above-described processes, the session process of step S140 of the operation flowchart of FIG. 4, i.e., the session process of one session for repeating the supply of the stimulation current to the electrode section 110 until a response is not confirmed, and obtaining the minimum current value that is the minimum stimulation current at which a response can be confirmed is ended.

Next, the controlling section 160 refers to the predetermined range of the effective current, and predetermined number of valid sessions that are stored in the storage section 165, and also to the minimum current values that are stored in the above-described session processes, and determines whether, within the range of the effective current, there are minimum current values the number of which is equal to or larger than the number of valid sessions or not (S150). Here, the range of the effective current means a range where minimum current values that are obtained in sessions correlate with one another, and the number of valid sessions means the number of minimum current values within the range of the effective current. For example, it is assumed that the range of the effective current is 0.1 mA and the number of valid sessions is 3. In step S150, it is determined whether minimum current values the number of which is equal to or larger than 3 sessions exist within the effective current range of 0.1 mA or not.

If there are not minimum current values the number of which is equal to or larger than the number of valid sessions, within the range of the effective current (S150: NO), the controlling section 160 refers to the predetermined number of sessions stored in the storage section 165, and determines whether the predetermined number of session processes are ended or not (S160).

If the predetermined number of session processes are not ended (S160: NO), the control returns to the process of step S110, and the controlling section 160 determines whether the process to be next performed is the first session process or not (S110). In this case, the process to be next performed is not the first session process (S110: NO), and therefore the controlling section 160 refers to the increment that is stored in the storage section 165, and that is used for the stimulation current that stepwise increases, and sets the stimulation current value of the next session (S130).

For example, it is assumed that 0.25 mA is stored as the minimum current value in the process of step S260 in the first session process (see FIG. 5). In the embodiment, the increment of the stimulation current is 0.1 mA. In this case, therefore, the controlling section 160 sets 0.35 mA as the stimulation current value in the next session.

Then, the controlling section 160 performs the next session process by using the stimulation current value that is set in the process of step S130 (S140). The session process is executed in accordance with the processes of the operation flowcharts of FIGS. 5 and 6. The controlling section 160 repeats the processes of steps S130 to S150 until the predetermined number of session processes are ended.

If the predetermined number of session processes are ended (S160: YES), by contrast, this means that, even when the predetermined number of session processes are repeated, there are not minimum current values the number of which is equal to or larger than the number of valid sessions, within the range of the effective current, and therefore the controlling section 160 causes information indicating that the sensitivity threshold cannot be measured, to be displayed on the displaying section 154 (S170). Although the burden on the subject is increased, a configuration may be possible where the processes are not ended when the predetermined number of session processes are performed, but the processes are continued until the sensitivity threshold can be calculated. Namely, the above-described processes of steps S160 and S170 may not be performed, and, if there are not minimum current values the number of which is equal to or larger than the number of valid sessions, within the range of the effective current (S150: NO), the series of processes (S130, S140, S150, and S180) may be repeated until the sensitivity threshold can be calculated.

Returning to the process of step S150, if there are minimum current values the number of which is equal to or larger than the number of valid sessions, within the range of the effective current (S150: YES), the controlling section 160 obtains the sensitivity threshold based on the minimum current value that is among the minimum current values of the sessions, and that is within the range of the effective current, and causes the sensitivity threshold to be displayed on the displaying section 154 (S180). The controlling section 160 sets, as the sensitivity threshold, the minimum or maximum value of all minimum current values that are within the range of the effective current in the number of valid sessions, or the average or median value of the all minimum current values. In the embodiment, the minimum value of all minimum current values that are within the range of the effective current in the number of valid sessions is set as the sensitivity threshold.

The series of operations of the nerve stimulation measurement device 100, the nerve stimulation measurement method, and the nerve stimulation measurement program have been described above. While exemplarily showing specific numerical values, next, the series of operations of the nerve stimulation measurement device 100, the nerve stimulation measurement method, and the nerve stimulation measurement program will be specifically described by dividing the operations into Processing mode 1 to Processing mode 4.

### < Processing mode 1>

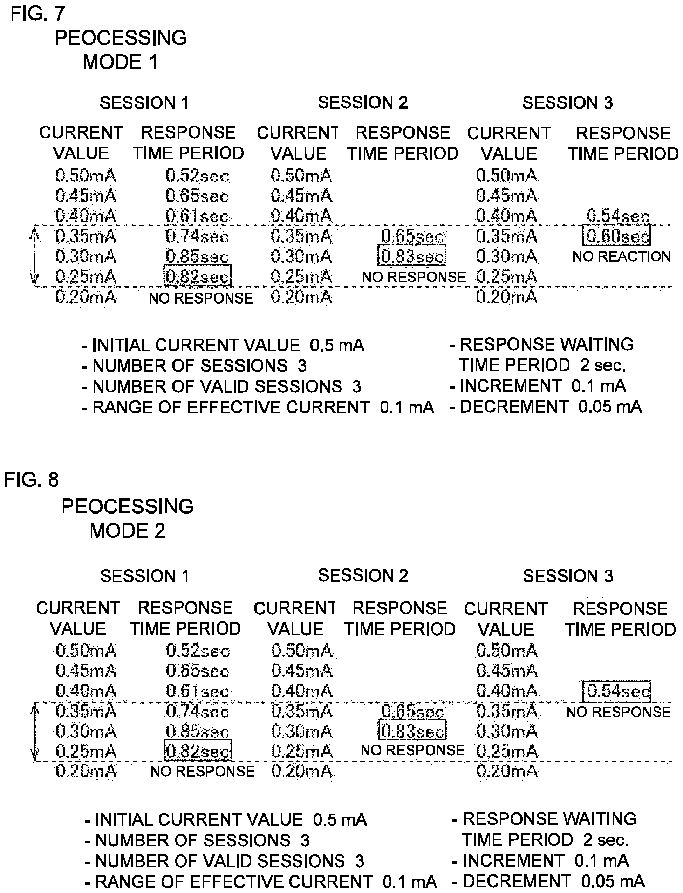

FIG. 7 is a view illustrating Processing mode 1 in the nerve stimulation measurement device of the embodiment. In Processing mode 1, 0.5 mA is previously stored as the initial current value, 3 as the predetermined number of sessions, 3 as the predetermined number of valid sessions, 0.1 mA as the predetermined range of the effective current, 2 sec. as the response waiting time period, 0.1 mA as the increment, and 0.05 mA as the decrement, in the storage section 165.

The controlling section 160 executes the process (the operation flowcharts of FIGS. 5 and 6) of the first session, i.e., session 1. First, the controlling section 160 sets 0.5 mA of the initial current value as the initial stimulation current value, and supplies the stimulation current of the initial stimulation current value to the electrode section 110. When the subject senses the electrical stimulation, the subject presses the response button 122 of the responding section 120. This causes the controlling section 160 to confirm the response. In the case of Processing mode 1, the time period from the supply of the stimulation current to the confirmation of the response, i.e., the response time period is 0.52 sec., and the response is confirmed within the response waiting time period of 2 sec. Therefore, the controlling section 160 determines that there is a response. Namely, it is determined that the subject can sense the stimulation current of 0.5 mA.

Then, the controlling section 160 refers to the decrement of the stimulation current, and sets the next stimulation current value. Since the decrement is 0.05 mA, the next stimulation current value is 0.5 - 0.05 = 0.45 mA. The controlling section 160 supplies the stimulation current of the stimulation current value to the electrode section 110. At this time, the response time period is 0.65 sec., and therefore the controlling section 160 determines that there is a response. Namely, it is determined that the subject can sense the stimulation current of 0.45 mA.

Then, the controlling section 160 checks the situation of a response performed by the subject while performing a process that is same as or similar to the above-described process, to sequentially decrease the stimulation current value by a step of 0.05 mA, or to 0.40 mA, 0.35 mA, 0.30 mA, 0.25 mA, and 0.2 mA. In the case of Processing mode 1, when the stimulation current value is 0.20 mA, the controlling section 160 determines that there is not a response. In other words, it is determined that the subject cannot sense the stimulation current of 0.20 mA. Therefore, the controlling section 160 knows that the subject can sense the stimulation current of 0.25 mA, but cannot sense that of 0.20 mA. The controlling section 160 causes 0.25 mA to be stored in the storage section 165 as the minimum current value of session 1.

Next, the controlling section 160 executes the process of session 2. When the process of session 2 is to be started, the controlling section 160 refers the increment of the stimulation current, and sets the first stimulation current value of session 2. Since the increment is 0.10 mA, the first stimulation current value is 0.25 + 0.10 = 0.35 mA. As described above, the process of session 2 is designed so as not to be started from 0.5 mA that is the initial stimulation current value of session 1, and therefore the measurement time period can be shortened.

The controlling section 160 starts a process that is same as or similar to the process in session 1, at the stimulation current value of 0.35 mA, and checks the situation of a response performed by the subject while sequentially decreasing the stimulation current value by a step of 0.05 mA, or to 0.30 mA and 0.25 mA. In the case of session 2, when the stimulation current value is 0.25 mA, the controlling section 160 determines that there is not a response. In other words, it is determined that, in session 2, the subject cannot sense the stimulation current of 0.25 mA. Therefore, the controlling section 160 knows that the subject can sense the stimulation current of 0.30 mA, but cannot sense that of 0.25 mA. The controlling section 160 causes 0.30 mA to be stored in the storage section 165 as the minimum current value of session 2.

Next, the controlling section 160 executes the process of session 3. The process of session 3 is identical with that of session 2. The controlling section 160 sets the first stimulation current value in session 3 as 0.30 + 0.10 = 0.40 mA, starts a process that is at the stimulation current of 0.40 mA, and that is same as or similar to the process in session 1, and checks the situation of a response performed by the subject while sequentially decreasing the stimulation current value by a step of 0.05 mA, or to 0.35 mA and 0.30 mA. In the case of session 3, when the stimulation current value is 0.30 mA, the controlling section 160 determines that there is not a response. In other words, it is determined that, in session 3, the subject cannot sense the stimulation current of 0.30 mA. Therefore, the controlling section 160 knows that the subject can sense the stimulation current of 0.35 mA, but cannot sense that of 0.30 mA. The controlling section 160 causes 0.35 mA to be stored in the storage section 165 as the minimum current value of session 3.

In the case of Processing mode 1, 3 is set as the predetermined number of sessions, and therefore the controlling section 160 ends the measurement of the minimum current value up to the process of session 3. In the processes of sessions 1 to 3, three minimum current values, i.e., 0.25 mAin session 1, 0.30 mA in session 2, and 0.35 mAin session 3 are measured.

Next, the controlling section 160 refers to the predetermined range of the effective current, and predetermined number of valid sessions that are stored in the storage section 165, and also to the minimum current values that are stored in above-described sessions 1 to 3, and determines whether, within the range of the effective current, there are minimum current values the number of which is equal to or larger than the number of valid sessions or not.

In the case of Processing mode 1, the range of the effective current is 0.1 mA, the number of valid sessions is 3, the stored 3 minimum current values are 0.25 mA, 0.30 mA, and 0.35 mA, and all of the 3 minimum current values are within the effective current range of 0.1 mA. Namely, there are minimum current values the number of which is equal to or larger than the number of valid sessions, within the range of the effective current. Therefore, the controlling section 160 causes the stimulation current that is minimum in the minimum currents of sessions 1 to 3, to be displayed on the displaying section 154 as the sensitivity threshold. In the case of Processing mode 1, 0.25 mA that is minimum in the minimum currents of sessions 1 to 3 is displayed on the displaying section 154 as the sensitivity threshold. The controlling section 160 may set, as the sensitivity threshold, the maximum value (in the above-described case, 0.35 mA) of all minimum current values that are within the range of the effective current in the number of valid sessions, or the average (in the above-described case, 0.30 mA) or median value (in the above-described case, 0.30 mA) of the all minimum current values.

### <Processing mode 2>

FIG. 8 is a view illustrating Processing mode 2 in the nerve stimulation measurement device of the embodiment. In Processing mode 2, in the same or similar manner as Processing mode 1, 0.5 mA is previously stored as the initial current value, 3 as the predetermined number of sessions, 3 as the predetermined number of valid sessions, 0.1 mA as the predetermined range of the effective current, 2 sec. as the response waiting time period, 0.1 mA as the increment, and 0.05 mA as the decrement, in the storage section 165.

In Processing mode 2, the processes of sessions 1 to 3 are executed in the procedure that is same as or similar to that of Processing mode 1. In the case of Processing mode 2, namely, 3 is set as the predetermined number of sessions, and therefore the controlling section 160 measures the minimum current values up to the process of session 3. In the processes of sessions 1 to 3, three minimum current values, i.e., 0.25 mA in session 1, 0.30 mA in session 2, and 0.40 mA in session 3 are measured.

In the same or similar manner as Processing mode1, next, the controlling section 160 refers to the predetermined range of the effective current, and predetermined number of valid sessions that are stored in the storage section 165, and also to the minimum current values that are stored in above-described processes of sessions 1 to 3, and determines whether, within the range of the effective current, there are minimum current values the number of which is equal to or larger than the number of valid sessions or not.

In the case of Processing mode 2, the range of the effective current is 0.1 mA, the number of valid sessions is 3, the stored 3 minimum current values are 0.25 mA, 0.30 mA, and 0.40 mA, and only two of the 3 minimum current values of sessions 1 to 3 are within the effective current range of 0.1 mA. Namely, there are not minimum current values the number of which is equal to or larger than the number of valid sessions, within the range of the effective current. Therefore, the controlling section 160 causes information indicating that the sensitivity threshold cannot be measured, to be displayed on the displaying section 154.

### <Processing mode 3>

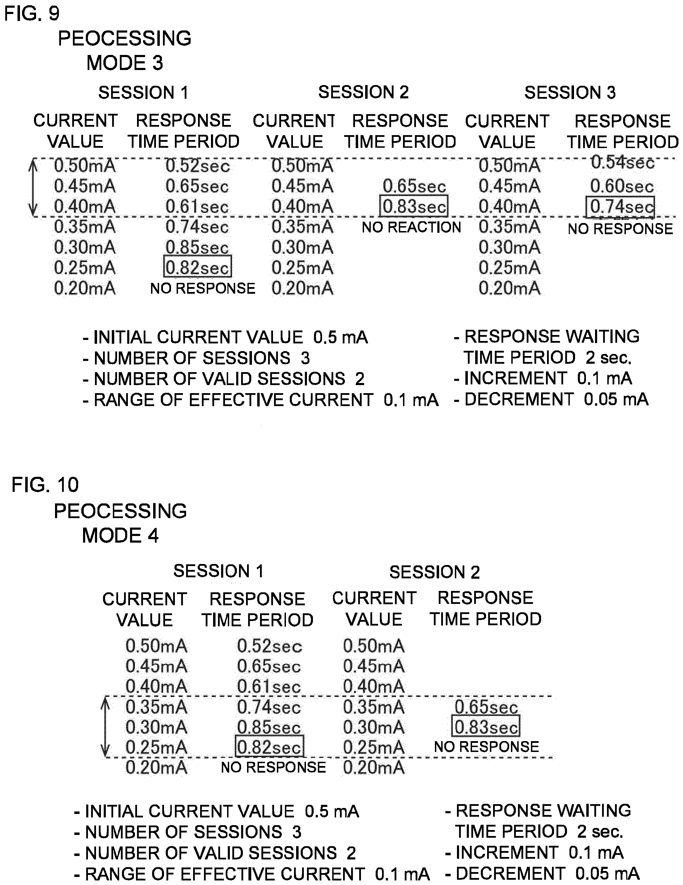

FIG. 9 is a view illustrating Processing mode 3 in the nerve stimulation measurement device of the embodiment. In Processing mode 3, 0.5 mA is previously stored as the initial current value, 3 as the predetermined number of sessions, 2 as the predetermined number of valid sessions, 0.1 mA as the predetermined range of the effective current, 2 sec. as the response waiting time period, 0.1 mA as the increment, and 0.05 mA as the decrement, in the storage section 165.

In Processing mode 3, the processes of sessions 1 to 3 are executed in the procedure that is same as or similar to that of Processing mode 1. In the case of Processing mode 3, namely, 3 is set as the predetermined number of sessions, and therefore the controlling section 160 ends the measurement of the minimum current value up to the process of session 3. In the processes of sessions 1 to 3, three minimum current values, i.e., 0.25 mA in session 1, 0.40 mA in session 2, and 0.40 mA in session 3 are measured.

In the same or similar manner as Processing mode1, next, the controlling section 160 refers to the predetermined range of the effective current, and predetermined number of valid sessions that are stored in the storage section 165, and also to the minimum current values that are stored in above-described sessions 1 to 3, and determines whether, within the range of the effective current, there are minimum current values the number of which is equal to or larger than the number of valid sessions or not.

In the case of Processing mode 3, the range of the effective current is 0.1 mA, the number of valid sessions is 2, the stored 3 minimum current values are 0.25 mA, 0.40 mA, and 0.40 mA, and 2 minimum current values (sessions 2 and 3) among the 3 minimum current values in sessions 1 to 3 are within the effective current range of 0.1 mA. Namely, there are minimum current values the number of which is equal to or larger than the number of valid sessions, within the range of the effective current. Therefore, the controlling section 160 causes 0.40 mA that is minimum in the minimum current values of sessions 2 and 3, to be displayed on the displaying section 154 as the sensitivity threshold.

### <Processing mode 4>

FIG. 10 is a view illustrating Processing mode 4 in the nerve stimulation measurement device of the embodiment. In Processing mode 4, in the same or similar manner as Processing mode 3, 0.5 mA is previously stored as the initial current value, 3 as the predetermined number of sessions, 2 as the predetermined number of valid sessions, 0.1 mA as the predetermined range of the effective current, 2 sec. as the response waiting time period, 0.1 mA as the increment, and 0.05 mA as the decrement, in the storage section 165.

In Processing mode 4, the session process is repeated, and, if it is determined that there are minimum stimulation current values the number of which is equal to or larger than the number of valid sessions, within the range of the effective current, the measurement performed by the controlling section 160 is ended at this timing, even when session processes of the predetermined session number are not completed. In Processing mode 4, therefore, the measurement time period is sometimes shorter as compared to Processing modes 1 to 3.

In Processing mode 4, the process of session 1 is executed in the procedure that is same or similar to that of Processing mode 1. Namely, first, the controlling section 160 sets 0.5 mA that is the initial current value, as the initial stimulation current value, and measures the minimum current value while the stimulation current value is sequentially decreased by a step of 0.05 mA. As a result of the measurement, 0.25 mA is measured as the minimum current value in session 1.

Then, the process of session 2 is performed. Namely, the controlling section 160 sets 0.35 mA as the next initial stimulation current value, and measures the minimum current value while sequentially decreasing the stimulation current value by a step of 0.05 mA. As a result of the measurement, 0.30 mA is measured as the minimum current value in session 2.

Since the predetermined number of valid sessions that is stored in the storage section 165 is 2, the controlling section 160 then determines whether the minimum current values that are measured in sessions 1 and 2 are within the effective current range of 0.1 mA or not. Since the minimum current values that are measured in sessions 1 and 2 are 0.25 mA and 0.30 mA, and the minimum current values are within the effective current range of 0.1 mA, the controlling section 160 does not perform the measurement process of session 3, and causes 0.25 mA that is minimum in the minimum currents that are within the effective current range, to be displayed on the displaying section 154 as the sensitivity threshold. In the case where the number of the minimum current values that are within the range of the effective current is equal to or larger than the number of valid sessions, when the execution of the following sessions is stopped, the inspection is completed in a short time period.

According to the nerve stimulation measurement device, nerve stimulation measurement method, and nerve stimulation measurement program of the above-described embodiment, the controlling section 160 is caused to automatically measure the sensitivity threshold, simply by pressing the start switch 152, and therefore the labor of the inspector is saved. Moreover, the timing when electrical stimulation is applied does not depend on the inspector, and therefore it is possible to obtain a reproducible measurement result.

Although the nerve stimulation measurement device, nerve stimulation measurement method, and nerve stimulation measurement program of the presently disclosed subject matter have been described by way of the embodiment, the technical scope of the nerve stimulation measurement device, nerve stimulation measurement method, and nerve stimulation measurement program of the presently disclosed subject matter is not restricted to the scope of the description of the embodiment. It is a matter of course that all embodiments that can be modified by those skilled in the art within the technical scope are included in the technical scope, as defined by the appended claims.

### REFERENCE SIGNS LIST

- 100: nerve stimulation measurement device,
- 110: electrode section,
- 115: cord,
- 120: responding section,
- 122: response button,
- 125: cord,
- 150: body unit,
- 152: start switch,
- 154: displaying section,
- 156: inputting section,
- 160: controlling section,
- 162: CPU,
- 164: RAM,
- 165: storage section,
- 166: ROM,
- 168: external I/F.

## Claims

1. A nerve stimulation measurement device (100) comprising:
a responding section (120) being configured to perform a response operation when a subject to whom an electrode section (110) that applies electrical stimulation is attached senses the electrical stimulation; and
a controlling section (160) being configured to supply a stimulation current which stepwise increases or decreases, to the electrode section, and being configured to obtain a sensitivity threshold that is a stimulation current from which the subject senses the electrical stimulation, from a situation of response to each stepwise supply,
wherein in a case where a stimulation current which stepwise increases is to be supplied to the electrode section, the controlling section stepwise increases the stimulation current according to a predetermined increment, and,
wherein in a case where a stimulation current which stepwise decreases is to be supplied to the electrode section, the controlling section stepwise decreases the stimulation current according to a predetermined decrement,
wherein the controlling section is configured to perform a session process in which the supply of the stimulation current to the electrode section is repeated until a response is not confirmed, and obtains a minimum current value that is a minimum stimulation current at which the response can be confirmed, and,
wherein in a case where a range where minimum current values that are obtained in sessions correlate with one another is set as a predetermined range of an effective current, and a number of sessions that are required for obtaining the sensitivity threshold is set as a number of valid sessions, when, within the predetermined range of the effective current, there are the minimum current values a number of which is equal to or larger than the predetermined number of valid sessions, the controlling section is configured to obtain the sensitivity threshold based on the minimum current values within the predetermined range of the effective current.

2. The nerve stimulation measurement device according to claim 1,
wherein the controlling section is configured to repeat the session process up to an upper limit that is equal to a maximum number of sessions that are executed in a predetermined number of sessions.

3. The nerve stimulation measurement device according to claim 1 or 2, wherein in a case where a number of the minimum current values that are within the predetermined range of the effective current is equal to or larger than the predetermined number of valid sessions, the controlling section is configured to stop execution of following sessions.

4. The nerve stimulation measurement device according to any one of claims 1 to 3, wherein in a case where a number of the minimum current values that are within the predetermined range of the effective current is not equal to or larger than the predetermined number of valid sessions, the controlling section is configured to determine that the measurement is impossible.

5. The nerve stimulation measurement device according to claim 2,
wherein the controlling section includes a storage section (165) that stores the predetermined increment, the predetermined decrement, the predetermined number of sessions, the predetermined number of valid sessions, and the predetermined range of the effective current.

6. The nerve stimulation measurement device according to claim 5, further comprises an inputting section (156),
wherein the predetermined increment, predetermined decrement, predetermined number of sessions, predetermined number of valid sessions, and predetermined range of the effective current that are to be stored in the storage section are input through the inputting section.

7. The nerve stimulation measurement device according to claim 4, further comprises a displaying section (154),
wherein the sensitivity threshold that is obtained by the controlling section, or information indicating that the measurement is impossible is displayed on the displaying section.

8. The nerve stimulation measurement device according to any one of claims 1 to 7, wherein the controlling section is configured to set a minimum or maximum value of all minimum current values that are within the predetermined range of the effective current, or an average or median value of the all minimum current values, as the sensitivity threshold.

9. The nerve stimulation measurement device according to any one of claims 1 to 8 further comprises a start switch (152),
wherein when the start switch is pressed, the controlling section is configured to automatically perform a series of processes from the supply of the stimulation current to the electrode section, to the obtaining of the sensitivity threshold.

10. A nerve stimulation measurement method including:
a step in which, when a subject to whom an electrode section (110) that applies electrical stimulation is attached senses the electrical stimulation, a responding section (120) performs a response operation; and
a step in which a controlling section (160) supplies a stimulation current which stepwise increases or decreases, to the electrode section, and obtains a sensitivity threshold that is a stimulation current from which the subject senses the electrical stimulation, from a situation of response to each stepwise supply,
wherein in a case where a stimulation current which stepwise increases is supplied to the electrode section, the controlling section stepwise increases the stimulation current according to a predetermined increment, and,
wherein in a case where a stimulation current which stepwise decreases is supplied to the electrode section, the controlling section stepwise decreases the stimulation current according to a predetermined decrement,
wherein the controlling section performs a session process in which the supply of the stimulation current to the electrode section is repeated until a response is not confirmed, and obtains a minimum current value that is a minimum stimulation current at which the response can be confirmed, and,
wherein in a case where a range where minimum current values that are obtained in sessions correlate with one another is set as a predetermined range of an effective current, and a number of sessions that are required for obtaining the sensitivity threshold is set as a number of valid sessions, when, within the predetermined range of the effective current, there are the minimum current values a number of which is equal to or larger than the predetermined number of valid sessions, the controlling section obtains the sensitivity threshold based on the minimum current values within the predetermined range of the effective current.

11. A nerve stimulation measurement program which causes a computer to execute:
a step in which, when a subject to whom an electrode section (110) that applies electrical stimulation is attached senses the electrical stimulation, a responding section (120) performs a response operation; and
a step in which a controlling section (160) supplies a stimulation current which stepwise increases or decreases, to the electrode section, and obtains a sensitivity threshold that is a stimulation current from which the subject senses the electrical stimulation, from a situation of response to each stepwise supply,
wherein in a case where a stimulation current which stepwise increases is supplied to the electrode section, the controlling section stepwise increases the stimulation current according to a predetermined increment, and,
wherein in a case where a stimulation current which stepwise decreases is supplied to the electrode section, the controlling section stepwise decreases the stimulation current according to a predetermined decrement,
wherein the controlling section performs a session process in which the supply of the stimulation current to the electrode section is repeated until a response is not confirmed, and obtains a minimum current value that is a minimum stimulation current at which the response can be confirmed, and,
wherein in a case where a range where minimum current values that are obtained in sessions correlate with one another is set as a predetermined range of an effective current, and a number of sessions that are required for obtaining the sensitivity threshold is set as a number of valid sessions, when, within the predetermined range of the effective current, there are the minimum current values a number of which is equal to or larger than the predetermined number of valid sessions, the controlling section obtains the sensitivity threshold based on the minimum current values within the predetermined range of the effective current.

## Patentansprüche

1. Vorrichtung (100) für Messung von Nervenstimulation, die umfasst:
einen reagierenden Abschnitt (120), der so ausgeführt ist, dass er einen Reaktionsvorgang durchführt, wenn ein Patient, an dem ein Elektrodenabschnitt (110) angebracht ist, der elektrische Stimulation ausübt, die elektrische Stimulation wahrnimmt; und
einen steuernden Abschnitt (160), der so ausgeführt ist, dass er dem Elektrodenabschnitt einen Stimulations-Strom zuführt, der schrittweise zunimmt oder abnimmt, und der so ausgeführt ist, dass er einen Empfindlichkeits-Schwellenwert, der ein Stimulations-Strom ist, ab dem der Patient die elektrische Stimulation wahrnimmt, anhand einer Situation von Reaktion auf jede schrittweise Zufuhr ermittelt,
wobei in einem Fall, in dem dem Elektrodenabschnitt ein schrittweise zunehmender Stimulations-Strom zugeführt werden soll, der steuernde Abschnitt den Stimulations-Strom entsprechend einem vorgegebenen Inkrement schrittweise erhöht, und
in einem Fall, in dem dem Elektrodenabschnitt ein schrittweise abnehmender Stimulations-Strom zugeführt werden soll, der steuernde Abschnitt den Stimulations-Strom entsprechend einem vorgegebenen Dekrement schrittweise verringert,
wobei der steuernde Abschnitt so ausgeführt ist, dass er einen Sitzungs-Prozess durchführt, bei dem die Zufuhr des Stimulations-Stroms zu dem Elektrodenabschnitt wiederholt wird, bis keine Reaktion bestätigt wird, und einen minimalen Stromwert ermittelt, der ein minimaler Stimulations-Strom ist, bei dem die Reaktion bestätigt werden kann, und
der steuernde Abschnitt so ausgeführt ist, dass er in einem Fall, in dem ein Bereich, in dem minimale Stromwerte, die in Sitzungen ermittelt werden, miteinander korrelieren, als ein vorgegebener Bereich eines effektiven Stroms eingestellt ist, und eine Anzahl von Sitzungen, die erforderlich sind, um den Empfindlichkeits-Schwellenwert zu ermitteln, als eine Anzahl gültiger Sitzungen eingestellt ist, wenn innerhalb des vorgegebenen Bereiches des effektiven Stroms die minimalen Stromwerte vorhanden sind, deren Anzahl genauso groß ist wie oder größer als die vorgegebene Anzahl gültiger Sitzungen, den Empfindlichkeits-Schwellenwert auf Basis der minimalen Stromwerte innerhalb des vorgegebenen Bereiches des effektiven Stroms ermittelt.

2. Vorrichtung für Messung von Nervenstimulation nach Anspruch 1,
wobei der steuernde Abschnitt so ausgeführt ist, dass er den Sitzungs-Prozess bis zu einer Obergrenze wiederholt, die einer maximalen Anzahl von Sitzungen entspricht, die innerhalb einer vorgegebenen Anzahl von Sitzungen ausgeführt werden.

3. Vorrichtung für Messung von Nervenstimulation nach Anspruch 1 oder 2, wobei der steuernde Abschnitt so ausgeführt ist, dass er in einem Fall, in dem eine Anzahl der minimalen Stromwerte, die innerhalb des vorgegebenen Bereiches des effektiven Stroms liegen, genauso groß ist wie oder größer als die vorgegebene Anzahl gültiger Sitzungen, Ausführung folgender Sitzungen abbricht.

4. Vorrichtung für Messung von Nervenstimulation nach einem der Ansprüche 1 bis 3, wobei der steuernde Abschnitt so ausgeführt ist, dass er in einem Fall, in dem eine Anzahl der minimalen Stromwerte, die innerhalb des vorgegebenen Bereiches des effektiven Stroms liegen, nicht genauso groß ist wie oder größer als die vorgegebene Anzahl gültiger Sitzungen, feststellt, dass die Messung nicht möglich ist.

5. Vorrichtung für Messung von Nervenstimulation nach Anspruch 2,
wobei der steuernde Abschnitt einen Speicherabschnitt (165) enthält, der das vorgegebene Inkrement, das vorgegebene Dekrement, die vorgegebene Anzahl von Sitzungen, die vorgegebene Anzahl gültiger Sitzungen sowie den vorgegebenen Bereich des effektiven Stroms speichert.

6. Vorrichtung für Messung von Nervenstimulation nach Anspruch 5, die des Weiteren einen Eingabeabschnitt (156) umfasst,
wobei das vorgegebene Inkrement, das vorgegebene Dekrement, die vorgegebene Anzahl von Sitzungen, die vorgegebene Anzahl gültiger Sitzungen und der vorgegebene Bereich des effektiven Stroms, die in dem Speicherabschnitt gespeichert werden sollen, über den Eingabeabschnitt eingegeben werden.

7. Vorrichtung für Messung von Nervenstimulation nach Anspruch 4, die des Weiteren einen Anzeigeabschnitt (154) umfasst,
wobei der von dem steuernden Abschnitt ermittelte Empfindlichkeits-Schwellenwert oder Informationen, die angeben, dass die Messung nicht möglich ist, auf dem Anzeigeabschnitt angezeigt werden.

8. Vorrichtung für Messung von Nervenstimulation nach einem der Ansprüche 1 bis 7, wobei der steuernde Abschnitt so ausgeführt ist, dass er einen minimalen oder einen maximalen Wert aller minimalen Stromwerte, die innerhalb des vorgegebenen Bereiches des effektiven Stroms liegen, oder einen durchschnittlichen oder mittleren Wert aller der minimalen Stromwerte als den Empfindlichkeits-Schwellenwert festlegt.

9. Vorrichtung für Messung von Nervenstimulation nach einem der Ansprüche 1 bis 8, die des Weiteren einen Start-Schalter (152) umfasst,
wobei der steuernde Abschnitt so ausgeführt ist, dass er, wenn der Start-Schalter gedrückt wird, automatisch eine Reihe von Prozessen von der Zufuhr des Stimulations-Stroms zu dem Elektrodenabschnitt bis zum Ermitteln des Empfindlichkeits-Schwellenwertes durchführt.

10. Verfahren für Messung von Nervenstimulation, das einschließt:
einen Schritt, in dem, wenn ein Patient, an dem ein Elektrodenabschnitt (110) angebracht ist, der elektrische Stimulation ausübt, die elektrische Stimulation wahrnimmt, ein reagierender Abschnitt (120) einen Reaktionsvorgang durchführt, sowie
einen Schritt, in dem ein steuernder Abschnitt (160) dem Elektrodenabschnitt einen Stimulations-Strom zuführt, der schrittweise zunimmt oder abnimmt, und einen Empfindlichkeits-Schwellenwert, der ein Stimulations-Strom ist, ab dem der Patient die elektrische Stimulation wahrnimmt, anhand einer Situation von Reaktion auf jede schrittweise Zufuhr ermittelt,
wobei in einem Fall, in dem dem Elektrodenabschnitt ein schrittweise zunehmender Stimulations-Strom zugeführt wird, der steuernde Abschnitt den Stimulations-Strom entsprechend einem vorgegebenen Inkrement schrittweise erhöht, und
in einem Fall, in dem dem Elektrodenabschnitt ein schrittweise abnehmender Stimulations-Strom zugeführt wird, der steuernde Abschnitt den Stimulations-Strom entsprechend einem vorgegebenen Dekrement schrittweise verringert,
wobei der steuernde Abschnitt einen Sitzungs-Prozess durchführt, bei dem die Zufuhr des Stimulations-Stroms zu dem Elektrodenabschnitt wiederholt wird, bis keine Reaktion bestätigt wird, und einen minimalen Stromwert ermittelt, der ein minimaler Stimulations-Strom ist, bei dem die Reaktion bestätigt werden kann, und
der steuernde Abschnitt in einem Fall, in dem ein Bereich, in dem minimale Stromwerte, die in Sitzungen ermittelt werden, miteinander korrelieren, als ein vorgegebener Bereich eines effektiven Stroms eingestellt ist, und eine Anzahl von Sitzungen, die erforderlich sind, um den Empfindlichkeits-Schwellenwert zu ermitteln, als eine Anzahl gültiger Sitzungen eingestellt ist, wenn innerhalb des vorgegebenen Bereiches des effektiven Stroms die minimalen Stromwerte vorhanden sind, deren Anzahl genauso groß ist wie oder größer als die vorgegebene Anzahl gültiger Sitzungen, den Empfindlichkeits-Schwellenwert auf Basis der minimalen Stromwerte innerhalb des vorgegebenen Bereiches des effektiven Stroms ermittelt.

11. Programm für Messung von Nervenstimulation, das einen Computer veranlasst, auszuführen:
einen Schritt, in dem, wenn ein Patient, an dem ein Elektrodenabschnitt (110) angebracht ist, der elektrische Stimulation ausübt, die elektrische Stimulation wahrnimmt, ein reagierenden Abschnitt (120) einen Reaktionsvorgang durchführt, sowie
einen Schritt, in dem ein steuernder Abschnitt (160) dem Elektrodenabschnitt einen Stimulations-Strom zuführt, der schrittweise zunimmt oder abnimmt, und einen Empfindlichkeits-Schwellenwert, der ein Stimulations-Strom ist, ab dem der Patient die elektrische Stimulation wahrnimmt, anhand einer Situation der Reaktion auf jede schrittweise Zufuhr ermittelt,
wobei in einem Fall, in dem dem Elektrodenabschnitt ein schrittweise zunehmender Stimulations-Strom zugeführt wird, der steuernde Abschnitt den Stimulations-Strom entsprechend einem vorgegebenen Inkrement schrittweise erhöht, und
in einem Fall, in dem dem Elektrodenabschnitt ein schrittweise abnehmender Stimulations-Strom zugeführt wird, der steuernde Abschnitt den Stimulations-Strom entsprechend einem vorgegebenen Dekrement schrittweise verringert,
wobei der steuernde Abschnitt einen Sitzungs-Prozess durchführt, bei dem die Zufuhr des Stimulations-Stroms zu dem Elektrodenabschnitt wiederholt wird, bis keine Reaktion bestätigt wird, und einen minimalen Stromwert ermittelt, der ein minimaler Stimulations-Strom ist, bei dem die Reaktion bestätigt werden kann, und
der steuernde Abschnitt in einem Fall, in dem ein Bereich, in dem minimale Stromwerte, die in Sitzungen ermittelt werden, miteinander korrelieren, als ein vorgegebener Bereich eines effektiven Stroms eingestellt ist, und eine Anzahl von Sitzungen, die erforderlich sind, um den Empfindlichkeits-Schwellenwert zu ermitteln, als eine Anzahl gültiger Sitzungen eingestellt ist, wenn innerhalb des vorgegebenen Bereiches des effektiven Stroms die minimalen Stromwerte vorhanden sind, deren Anzahl genauso groß ist wie oder größer als die vorgegebene Anzahl gültiger Sitzungen, den Empfindlichkeits-Schwellenwert auf Basis der minimalen Stromwerte innerhalb des vorgegebenen Bereiches des effektiven Stroms ermittelt.

## Revendications

1. Dispositif de mesure de stimulation de nerf (100) comprenant :
une section de réponse (120) conçue pour exécuter une opération de réponse lorsqu'un sujet, auquel une section d'électrode (110) qui applique une stimulation électrique est fixée, détecte la stimulation électrique ; et
une section de commande (160) conçue pour alimenter un courant de stimulation qui croît ou décroît progressivement, à la section d'électrode, et conçue pour obtenir un seuil de sensibilité qui est un courant de stimulation à partir duquel le sujet détecte la stimulation électrique, à partir d'une situation de réponse à chaque alimentation progressive,
dans un cas où un courant de stimulation qui croît progressivement doit être alimenté à la section d'électrode, la section de commande fait croître progressivement le courant de stimulation en fonction d'un pas prédéterminé, et,
dans un cas où un courant de stimulation qui décroît progressivement doit être alimenté à la section d'électrode, la section de commande fait décroître progressivement le courant de stimulation en fonction d'une diminution prédéterminée,
la section de commande étant conçue pour exécuter un processus par session dans lequel l'alimentation du courant de stimulation à la section d'électrode est répétée tant qu'une réponse n'est pas confirmée, et obtient une valeur de courant minimale qui est un courant de stimulation minimal auquel la réponse peut être confirmée, et,
dans un cas où une plage, où des valeurs de courant minimales qui sont obtenues dans des sessions sont corrélées les unes aux autres, est définie comme plage prédéterminée d'un courant efficace, et qu'un nombre de sessions qui sont requises pour obtenir le seuil de sensibilité est défini comme nombre de sessions valides, lorsque, à l'intérieur de la plage prédéterminée du courant efficace, il existe des valeurs de courant minimales dont un nombre est supérieur ou égal au nombre prédéterminé de sessions valides, la section de commande est conçue pour obtenir le seuil de sensibilité sur la base des valeurs de courant minimales à l'intérieur de la plage prédéterminée du courant efficace.

2. Dispositif de mesure de stimulation de nerf selon la revendication 1,
la section de commande étant conçue pour répéter le processus par session jusqu'à une limite supérieure qui est égale à un nombre maximal de sessions qui sont exécutées dans un nombre prédéterminé de sessions.

3. Dispositif de mesure de stimulation de nerf selon la revendication 1 ou 2, dans un cas où un nombre de valeurs de courant minimales qui se situent à l'intérieur de la plage prédéterminée du courant efficace est supérieur ou égal au nombre prédéterminé de sessions valides, la section de commande est conçue pour stopper l'exécution des sessions suivantes.

4. Dispositif de mesure de stimulation de nerf selon l'une quelconque des revendications 1 à 3, dans un cas où un nombre des valeurs de courant minimales qui se situent à l'intérieur de la plage prédéterminée du courant efficace n'est pas supérieur ou égal au nombre prédéterminé de sessions valides, la section de commande est conçue pour déterminer que la mesure est impossible.

5. Dispositif de mesure de stimulation de nerf selon la revendication 2,
la section de commande incluant une section de stockage (165) qui stocke le pas prédéterminé, la diminution prédéterminée, le nombre prédéterminé de sessions, le nombre prédéterminé de sessions valides, et la plage prédéterminée du courant efficace.

6. Dispositif de mesure de stimulation de nerf selon la revendication 5, comprenant en outre une section d'entrée (156),
le pas prédéterminé, la diminution prédéterminée, le nombre prédéterminé de sessions, le nombre prédéterminé de sessions valides, et la plage prédéterminée du courant efficace qui doivent être stockés dans la section de stockage sont saisis à travers la section d'entrée.

7. Dispositif de mesure de stimulation de nerf selon la revendication 4, comprenant en outre une section d'affichage (154),
le seuil de sensibilité qui est obtenu par la section de commande, ou les informations indiquant que la mesure est impossible étant affiché·es sur la section d'affichage.

8. Dispositif de mesure de stimulation de nerf selon l'une quelconque des revendications 1 à 7, la section de commande étant conçue pour définir une valeur minimale ou maximale de toutes les valeurs de courant minimales qui se situent à l'intérieur de la plage prédéterminée du courant efficace, ou une valeur moyenne ou médiane de toutes les valeurs de courant minimales, comme seuil de sensibilité.

9. Dispositif de mesure de stimulation de nerf selon l'une quelconque des revendications 1 à 8 comprenant en outre un commutateur de démarrage (152),
lorsque le commutateur de démarrage est pressé, la section de commande est conçue pour exécuter automatiquement une série de processus allant de l'alimentation du courant de stimulation à la section d'électrode, à l'obtention du seuil de sensibilité.

10. Procédé de mesure de stimulation de nerf incluant :
une étape dans laquelle, lorsqu'un sujet auquel une section d'électrode (110) qui applique une stimulation électrique est fixée détecte la stimulation électrique, une section de réponse (120) exécute une opération de réponse ; et
une étape dans laquelle une section de commande (160) alimente un courant de stimulation qui croît ou décroît progressivement, à la section d'électrode, et obtient un seuil de sensibilité qui est un courant de stimulation à partir duquel le sujet détecte la stimulation électrique, à partir d'une situation de réponse à chaque alimentation progressive,
dans un cas où un courant de stimulation qui croît progressivement est alimenté à la section d'électrode, la section de commande fait croître progressivement le courant de stimulation en fonction d'un pas prédéterminé, et,
dans un cas où un courant de stimulation qui décroît progressivement est alimenté à la section d'électrode, la section de commande fait décroître progressivement le courant de stimulation en fonction d'une diminution prédéterminée,
la section de commande exécutant un processus par session dans lequel l'alimentation du courant de stimulation à la section d'électrode est répétée tant qu'une réponse n'est pas confirmée, et obtient une valeur de courant minimale qui est un courant de stimulation minimal auquel la réponse peut être confirmée, et,
dans un cas où une plage où des valeurs de courant minimales qui sont obtenues dans des sessions sont corrélées les unes aux autres est définie comme plage prédéterminée d'un courant efficace, et qu'un nombre de sessions qui sont requises pour obtenir le seuil de sensibilité est défini comme nombre de sessions valides, lorsque, à l'intérieur de la plage prédéterminée du courant efficace, il existe des valeurs de courant minimales dont un nombre est supérieur ou égal au nombre prédéterminé de sessions valides, la section de commande obtient le seuil de sensibilité sur la base des valeurs de courant minimales à l'intérieur de la plage prédéterminée du courant efficace.

11. Programme de mesure de stimulation de nerf qui amène un ordinateur à exécuter :
une étape dans laquelle, lorsqu'un sujet auquel une section d'électrode (110) qui applique une stimulation électrique est fixée détecte la stimulation électrique, une section de réponse (120) exécute une opération de réponse ; et
une étape dans laquelle une section de commande (160) alimente un courant de stimulation qui croît ou décroît progressivement, à la section d'électrode, et obtient un seuil de sensibilité qui est un courant de stimulation à partir duquel le sujet détecte la stimulation électrique, à partir d'une situation de réponse à chaque alimentation progressive,
dans un cas où un courant de stimulation qui croît progressivement est alimenté à la section d'électrode, la section de commande fait croître progressivement le courant de stimulation en fonction d'un pas prédéterminé, et,
dans un cas où un courant de stimulation qui décroît progressivement est alimenté à la section d'électrode, la section de commande fait décroître progressivement le courant de stimulation en fonction d'une diminution prédéterminée,
la section de commande exécute un processus par session dans lequel l'alimentation du courant de stimulation à la section d'électrode est répétée tant qu'une réponse n'est pas confirmée, et obtient une valeur de courant minimale qui est un courant de stimulation minimal auquel la réponse peut être confirmée, et,
dans un cas où une plage où des valeurs de courant minimales qui sont obtenues dans des sessions sont corrélées les unes aux autres est définie comme plage prédéterminée d'un courant efficace, et qu'un nombre de sessions qui sont requises pour obtenir le seuil de sensibilité est défini comme nombre de sessions valides, lorsque, à l'intérieur de la plage prédéterminée du courant efficace, il existe des valeurs de courant minimales dont un nombre est supérieur ou égal au nombre prédéterminé de sessions valides, la section de commande obtient le seuil de sensibilité sur la base des valeurs de courant minimales à l'intérieur de la plage prédéterminée du courant efficace.
